# FASCICULE DE BREVET EUROPEEN

(11) **EP 1 289 987 B1**
(45) Date de publication et mention de la délivrance du brevet: **19.05.2004**
(21) Numéro de dépôt: 01938362.9
(22) Date de dépôt: 29.05.2001
(51) Int. Cl.: C07D 471/08, A61K 31/55, A61P 25/00

(54) **DERIVES DE 4-(2-PHENYLTHIAZOL-5-YL)-1,4-DIAZABICYCLO- 3.2.2]NONANE, LEUR PREPARATION ET LEUR APPLICATION EN THERAPEUTIQUE**
4-(2-PHENYLTHIAZOL-5-YL)1,4-DIAZABICYCLO[3.2.2]NONANEDERIVATE, IHRE HERSTELLUNG UND THERAPEUTISCHE VERWENDUNG
4-(2-PHENYLTHIAZOL-5-YL)-1,4-DIAZABICYCLO- 3.2.2]NONANE DERIVATIVES, PREPARATION AND THERAPEUTIC USE THEREOF

(30) Priorité: 31.05.2000 FR 0006978
(43) Date de publication de la demande: 12.03.2003
(73) Titulaire: SANOFI-SYNTHELABO, 75013 Paris (FR)
(72) Inventeur: GALLET, Thierry, F-91120 Palaiseau (FR); GALLI, Frédéric, F-92420 Vaucresson (FR); LECLERC, Odile, F-91640 Briis sous Forges (FR); LOCHEAD, Alistair, F-94220 Charenton le Pont (FR)
(74) Mandataire: Ludwig, Jacques
(86) Numéro de dépôt international: PCT/FR2001/001650
(87) Numéro de publication internationale: WO 2001/092260

(56) Documents cités:
- EP-A- 0 307 140
- WO-A-00/34279
- US-A- 5 478 939

## Description

Les composés de la présente invention répondent à la formule générale (I) 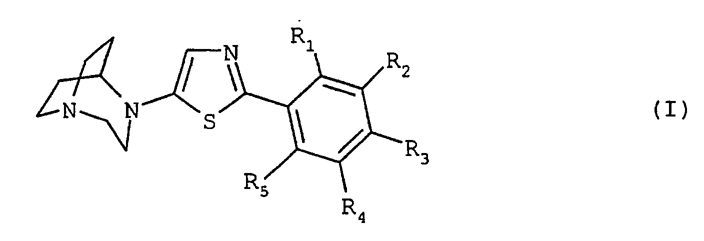 dans laquelle
R₁, R₂, R₃, R₄ et R₅ représentent chacun, indépendamment les uns des autres, un atome d'hydrogène ou d'halogène ou un groupe nitro, amino, trifluorométhyle, trifluoroalcoxy, cyano, hydroxy, (C₁-C₆)alkyle ou (C₁-C₆)alcoxy.

Les composés de l'invention peuvent exister à l'état de bases ou de sels d'addition à des acides.

Conformément à l'invention, on peut préparer les composés de formule générale (I) en faisant réagir le 1,4-diazabicyclo[3.2.2]nonane, de formule (II), 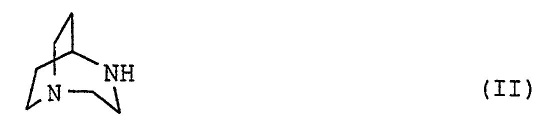 avec un composé de formule générale (III) 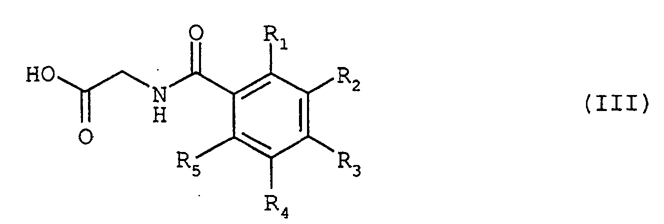 dans laquelle R₁, R₂, R₃, R₄ et R₅ sont tels que définis ci-dessus, puis on effectue une cyclisation de ce dernier en présence de dimère de sulfure de 4-méthoxyphénylthionophosphine (réactif de Lawesson), ou bien en présence de 2,4-disulfure de 2,4-bis(phénylthio)1,3,2,4-dithiadiphosphetane (*cf. Synt. Commun.* 1984, 827).

La préparation du 1,4-diazabicyclo[3.2.2]nonane est décrite dans *J. Med. Chem.* 1993, **36**, 2311-2320.

Les composés de formule générale (III) sont disponibles dans le commerce ou sont accessibles par des méthodes décrites dans la littérature.

Les exemples qui vont suivre illustrent la préparation de quelques composés selon l'invention. Les microanalyses élémentaires, et les spectres I.R. et R.M.N. confirment les structures des composés obtenus.
Les numéros indiqués entre parenthèses dans les titres des exemples correspondent à ceux de la 1ère colonne du tableau 1 donné plus loin.
Dans les noms des composés, le tiret "-" fait partie du mot, et le tiret "-" ne sert que pour la coupure en fin de ligne ; il est à supprimer en l'absence de coupure, et ne doit être remplacé ni par un tiret normal ni par un espace.

### Exemple 1 (Composé N°1).

### Bromhydrate de 4-(2-phénylthiazol-5-yl)-1,4-diazabicyclo[3.2.2]nonane 2:1.

### 1.1 N-[2-(1,4-Diazabicyclo[3.2.2]non-4-yl)-2-oxoéthyl]benzamide.

Dans un ballon de 50 ml on introduit 1,06 g (5,9 mmoles) de *N*-benzoylglycine (acide hippurique) en solution dans 20 ml de chloroforme et on ajoute 2,9 g (17,9 mmoles) de 1,1'-carbonyl-bis-1*H*-imidazole, puis on agite à température ambiante pendant 1 h.
On ajoute 0,75 g (5,9 mmoles) de 1,4-diazabicyclo[3.2.2]nonane en solution dans 5 ml de chloroforme et on agite pendant 24 h.
On évapore le solvant sous pression réduite et on purifie le résidu par chromatographie sur colonne de gel de silice en éluant avec un mélange 90/10/1 de chloroforme, méthanol et ammoniaque.
On obtient 1,3 g de produit sous forme d'huile.

### 1.2. Bromhydrate de 4-(2-phénylthiazol-5-yl)-1,4-diazabicyclo[3.2.2]nonane 2:1.

Dans un ballon de 100 ml on introduit 1,3 g (4,5 mmoles)de *N-*[2-(1,4-diazabicyclo[3.2.2]non-4-yl)-2-oxoéthyl]benzamide en solution dans 50 ml de toluène, on ajoute 1,8 g (4,5 mmoles) de dimère de sulfure de 4-méthoxyphénylthionophosphine (réactif de Lawesson) et on chauffe le mélange à 120°C pendant 18 h.
On évapore le solvant sous pression réduite et on purifie le résidu par chromatographie sur colonne de gel de silice en éluant avec un mélange 95/5/0,5 de chloroforme, méthanol et ammoniaque. On dissout le produit obtenu dans l'alcool isopropylique et on ajoute une solution d'acide bromhydrique à 33% dans l'acide acétique. On collecte les cristaux obtenus (0,14 g) par filtration.
Point de fusion : 275-277°C.

### Exemple 2 (Composé N°5).

### Bromhydrate de 4-[2-(2-méthylphényl)thiazol-5-yl]-1,4-diazabicyclo[3.2.2]nonane 2:1.

### 2.1. N-[2-(1,4-Diazabicyclo[3.2.2]non-4-yl)-2-oxoéthyl]-2-méthylbenzamide.

Dans un ballon de 50 ml on introduit 0,47 g (2,28 mmoles) de dicyclohexylcarbodiimide en solution dans 20 ml de dioxane à température ambiante. On ajoute ensuite 0,4 g (2,07 mmoles) de *N*-(o-toluyl)glycine et agite pendant 30 min à température ambiante.
On ajoute 0,26 g (2,07 mmoles) de 1,4-diazabicyclo[3.2.2]nonane en solution dans 5 ml de dioxane et on agite le mélange pendant 1 h.
On ajoute 50 ml d'eau, on filtre le précipité formé et on extrait la phase aqueuse par du chloroforme. On extrait la phase organique par une solution aqueuse 0,1 N d'acide chlorhydrique, alcalinise la phase aqueuse jusqu'à pH 10 par addition d'une solution aqueuse concentrée d'hydroxyde de sodium et on l'extrait par du chloroforme.
On sèche la phase organique sur sulfate de sodium et on la concentre sous pression réduite.
On obtient 0,41 g de produit sous forme solide.
Point de fusion : 177°C.

### 2.2. Bromhydrate de 4-[2-(2-méthylphényl)thiazol-5-yl]-1,4-diazabicyclo[3.2.2]nonane 2:1.

Dans un ballon de 50 ml on introduit 0,41 g (1,36 mmoles) de *N*-[2-(1,4-diazabicyclo[3.2.2]non-4-yl)-2-oxoéthyl]-2-méthylbenzamide en suspension dans 20 ml de xylène, on ajoute 0,611 g (1,5 mmoles) de 2,4-disulfure de 2,4-bis(phénylthio)1,3,2,4-dithiadiphosphetane et on chauffe le mélange au reflux pendant 20 h.
On évapore le solvant sous pression réduite et on purifie le résidu par chromatographie sur colonne de gel de silice en éluant avec un mélange 95/5/0,5 de chloroforme, méthanol et ammoniaque.
On dissout le produit obtenu solution dans l'éthanol, on ajoute une solution d'acide bromhydrique à 33% dans l'acide acétique et on recristallise les cristaux obtenus dans l'alcool isopropylique.
On obtient 0,168 g de produit sous forme de solide.
Point de fusion : 276-279°C.

### Exemple 3 (Composé N°7).

### Oxalate de 4-[2-(3-méthoxyphényl)thiazol-5-yl]-1,4-diazabicyclo[3.2.2]nonane 1:1.

### 3.1. N-(3-Méthoxybenzoyl)glycine.

Dans un ballon de 50 ml on introduit 1,5 g (20 mmoles) de glycine en solution dans 20 ml d'une solution aqueuse 2N d'hydroxyde de sodium, on chauffe le milieu à 50°C, on ajoute, goutte à goutte, 3,1 ml (20 mmoles) de chlorure de 3-méthoxybenzoyle et on agite le mélange à 50°C pendant 30 min. On le refroidit à température ambiante et on maintient l'agitation pendant 20 h.
On refroidit le milieu réactionnel à 4°C et on ajoute lentement 2 ml d'une solution aqueuse concentrée d'acide chlorhydrique. On collecte le précipité obtenu par filtration et on le recristallise dans le toluène.
On obtient 2,77 g de cristaux.
Point de fusion : 124°C.

### 3.2. N-[2-(1,4-Diazabicyclo[3.2.2]non-4-yl)-2-oxoéthyl]-3-méthoxybenzamide.

Dans un ballon de 50 ml on introduit 0,42 g (2 mmoles) de *N*-(3-méthoxybenzoyl)glycine dans 20 ml de dioxane, on ajoute 0,45 g (2,2 mmoles) de dicyclohexylcarbodiimide, on agite le mélange à température ambiante pendant 30 min, on ajoute 0,25 g (2 mmoles) de 1,4-diazabicyclo[3.2.2]nonane et on agite encore pendant 2 h.
On ajoute 20 ml d'eau, on filtre le précipité formé et on extrait le filtrat avec du chloroforme. On extrait la phase organique avec une solution aqueuse 0,1 N d'acide chlorhydrique, on alcalinise la phase aqueuse d'extraction jusqu'à pH 10 par addition d'une solution aqueuse concentrée d'hydroxyde de sodium, et on l'extrait avec du chloroforme. On sèche la phase organique sur sulfate de sodium et on la concentre sous pression réduite.
On obtient 0,43 g de produit sous forme de solide amorphe.

### 3.3. Oxalate de 4-[2-(3-méthoxyphényl)thiazol-5-yl]-1,4-diazabicyclo[3.2.2]nonane 1:1.

Dans un ballon de 25 ml on introduit 0,42 g (1,32 mmoles) de *N*-[2-(1,4-diazabicyclo[3.2.2]non-4-yl)-2-oxoéthyl]-3-méthoxybenzamide en suspension dans 15 ml de xylène, on ajoute 0,59 g (1,45 mmoles) de 2,4-disulfure de 2,4-bis(phénylthio)1,3,2,4-dithiadiphosphetane et on chauffe le mélange au reflux pendant 20 h.
On ajoute une solution aqueuse 0,5N d'hydroxyde de sodium et on extrait la phase aqueuse par du chloroforme. On sèche la phase organique sur sulfate de sodium on l'évapore sous pression réduite et on purifie le résidu par chromatographie sur colonne de gel de silice en éluant avec un mélange 90/10 d'acétate d'éthyle et de méthanol. On dissout le produit obtenu dans l'acétone, on ajoute une solution d'acide oxalique dans l'acétone et on collecte les cristaux obtenus (0,166 g) par filtration.
Point de fusion : 192-193°C.

### Exemple 4 (Composé N°6).

### Oxalate de 4-[2-(4-méthoxyphényl)thiazol-5-yl]-1,4-diazabicyclo[3.2.2]nonane 1:1.

### 4.1. N-(4-Méthoxybenzoyl)glycine.

Dans un ballon de 250 ml on introduit 2,93 g (39 mmoles) de glycine en solution dans 41 ml d'une solution aqueuse 1N d'hydroxyde de sodium, on refroidi à 4°C, on ajoute simultanément 41 ml d'une solution aqueuse 1N d'hydroxyde de sodium et une solution de 7 g (0,041 mol) de chlorure de 4-méthoxybenzoyle dans 10 ml de dioxane, goutte à goutte en 45 min et on agite le mélange pendant 20 h.
On ajoute une solution aqueuse concentrée d'acide chlorhydrique jusqu'à pH 1, on recueille le précipité formé par filtration et on le recristallise dans l'alcool isopropylique.
On obtient 3,74 g de produit.
Point de fusion : 173°C.

### 4.2. N-[2-(1,4-Diazabicyclo[3.2.2]non-4-yl)-2-oxoéthyl]-4-méthoxybenzamide.

Dans un ballon de 50 ml on introduit 0,42 g (2 mmoles) de *N*-(4-méthoxybenzoyl)glycine en solution dans 20 ml de dioxane, on ajoute 0,45 g (2,2 mmoles) de dicyclohexylcarbodiimide, agite le mélange à température ambiante pendant 30 min, on ajoute 0,25 g (2 mmoles) de 1,4-diazabicyclo[3.2.2]nonane et on agite le mélange encore pendant 1 h.
On ajoute 20 ml d'eau, on filtre le précipité formé, on extrait le filtrat avec du chloroforme, on extrait la phase organique avec une solution aqueuse 0,1 N d'acide chlorhydrique, on alcalinise la phase aqueuse d'extraction jusqu'à pH 10 par addition d'une solution aqueuse concentrée d'hydroxyde de sodium et on l'extrait avec du chloroforme. On sèche la phase organique sur sulfate de sodium et on la concentre sous pression réduite.
On obtient 0,49 g de produit sous forme de solide amorphe.

### 4.3. Oxalate de 4-[2-(4-méthoxyphényl)thiazol-5-yl]-1,4-diazabicyclo[3.2.2]nonane 1:1.

Dans un ballon de 25 ml on introduit 0,47 g (1,48 mmoles) de *N*-[2-(1,4-diazabicyclo[3.2.2]non-4-yl)-2-oxoéthyl]-4-méthoxybenzamide en solution dans 15 ml de xylène, on ajoute 0,66 g (1,63 mmoles) de 2,4-disulfure de 2,4-bis(phénylthio)1,3,2,4-dithiadiphosphetane et on chauffe le mélange au reflux pendant 2 h.
On évapore le solvant sous pression réduite et on purifie le résidu par chromatographie sur colonne de gel de silice en éluant avec un mélange 95/5/0,5 d'acétate d'éthyle, méthanol et diéthylamine. On dissout le produit obtenu dans l'éthanol, on l'ajoute à une solution d'acide oxalique dans l'éthanol. On collecte les cristaux obtenus (0,176 g) par filtration.
Point de fusion : 219-222°C.

### Exemple 5 (Composé N°8).

### Oxalate de 4-[2-(3-bromophényl)thiazol-5-yl]-1,4-diazabicyclo[3.2.2]nonane 1:1.

### 5.1. N-(3-Bromobenzoyl)glycine.

Dans un ballon de 50 ml on introduit 1,5 g (20 mmoles) de glycine en solution dans 20 ml d'une solution aqueuse 2N d'hydroxyde de sodium. On chauffe le milieu à 50°C, on ajoute, goutte à goutte, 2,6 ml (20 mmoles) de chlorure de 3-bromobenzoyle, on agite le mélange pendant 30 min à cette température, on le refroidit à température ambiante et on l'agite pendant 20 h.
On refroidit le milieu réactionnel à 4°C, on ajoute lentement 2 ml d'une solution aqueuse concentrée d'acide chlorhydrique, on recueille le précipité obtenu par filtration et on le recristallise dans le toluène.
On obtient 3,21 g de cristaux.

### 5.2. N-[2-(1,4-Diazabicyclo[3.2.2]non-4-yl)-2-oxoéthyl]-3-bromobenzamide.

Dans un ballon de 100 ml on introduit 0,77 g (3 mmoles) de *N*-(3-bromobenzoyl)glycine en solution dans 30 ml de dioxane, on ajoute 0,68 g (3,3 mmoles) de dicyclohexylcarbodiimide, on agite le mélange à température ambiante pendant 30 min, on ajoute 0,38 g (3 mmoles) de 1,4-diazabicyclo[3.2.2]nonane et on agite le mélange encore pendant 2 h.
On ajoute 30 ml d'eau, on filtre le précipité formé, on extrait le filtrat avec du chloroforme puis on extrait la phase organique avec une solution aqueuse 0,1 N d'acide chlorhydrique. On alcalinise la phase aqueuse d'extraction jusqu'à pH 10 par addition d'une solution aqueuse concentrée d'hydroxyde de sodium et on l'extrait avec du chloroforme. On sèche la phase organique sur sulfate de sodium et on la concentre sous pression réduite.
On obtient 0,95 g de produit sous forme d'huile.

### 5.3. Oxalate de 4-[2-(3-bromophényl)thiazol-5-yl]-1,4-diazabicyclo[3.2.2]nonane 1:1.

Dans un ballon de 25 ml on introduit 0,93 g (2,54 mmoles) de *N*-[2-(1,4-diazabicyclo[3.2.2]non-4-yl)-2-oxoéthyl]-3-bromobenzamide en solution dans 25 ml de xylène, on ajoute 1,14 g (2,79 mmoles) de 2,4-disulfure de 2,4-bis(phénylthio)1,3,2,4-dithiadiphosphetane et on chauffe le mélange au reflux pendant 2 h.
On évapore le solvant sous pression réduite et on purifie le résidu par chromatographie sur colonne de gel de silice en éluant avec un mélange 95/5/0,5 d'acétate d'éthyle, méthanol et ammoniaque. On dissout le produit obtenu dans l'éthanol et on l'ajoute à une solution d'acide oxalique dans l'éthanol. On collecte les cristaux obtenus (0,055 g) par filtration.
Point de fusion : 161-163°C.

Le tableau 1 qui suit illustre les structures chimiques et les propriétés physiques de quelques composés de l'invention.

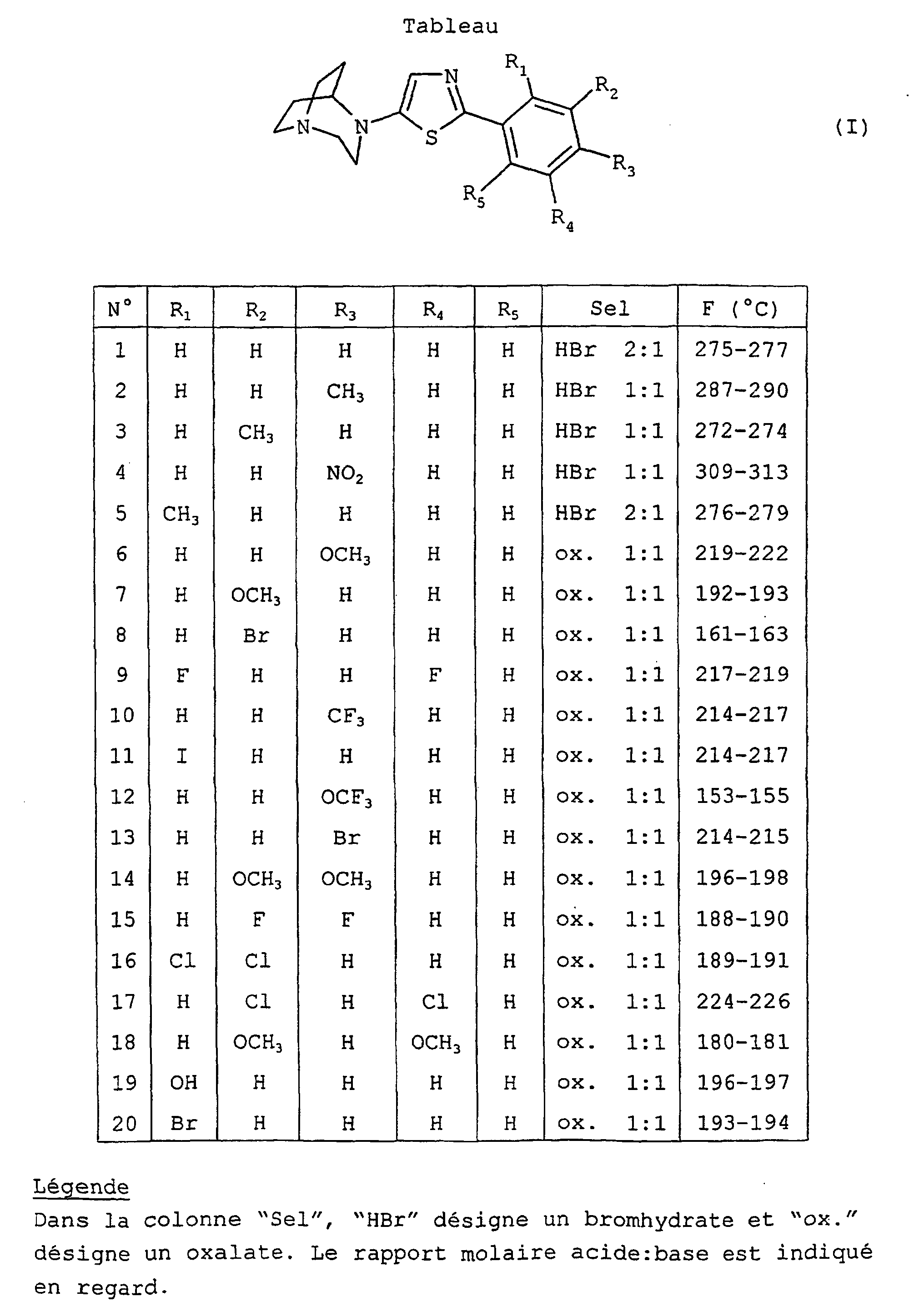

Les composés de l'invention ont fait l'objet d'essais qui ont mis en évidence leur intérêt comme substances thérapeutiques.

Ainsi ils ont été étudiés quant à leur affinité vis à vis des récepteurs nicotiniques contenant la sous unité α₇ selon les méthodes décrites par Marks et Collins dans *Mol. Pharmacol.* 1982, **22,** 554 et par Marks et coll. dans *Mol. Pharmacol.* 1986, **30,** 427.
On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4°C, puis on le centrifuge à 1000 g pendant 10 min. On élimine le culot et on centrifuge le surnageant à 8000 g pendant 20 min à 4 °C. On récupère le culot et on l'homogénéise à l'aide d'un broyeur Polytron™ dans 15 volumes d'eau bidistillée à 4°C, puis on le centrifuge à 8000 g pendant 20 min. On élimine le culot et on centrifuge le surnageant et la couche de peau ("buffy coat") à 40000 g pendant 20 min. On récupère le culot, on le remet en suspension avec 15 volumes d'eau bidistillée à 4°C et on le centrifuge encore une fois à 40000 g pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]α-bungarotoxine à 1 nM dans un volume final de 250 µl de tampon HEPES 20 mM. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM ; la liaison non spécifique représente environ 60% de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.

Les CI50 des composés de l'invention les plus affins se situent entre 0,020 et 0,500 µM.

Les composés de la présente invention ont aussi été étudiés quant à leur affinité vis-à-vis des récepteurs nicotiniques contenant la sous unité α₄β₂ selon les méthodes décrites par Anderson et Arneric dans *Eur. J. Pharmacol.* 1994, **253**, 261 et par Hall et coll. dans *Brain Res.* 1993, **600**, 127.
On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 g pendant 10 min. On élimine le culot et centrifuge le surnageant à 20000 g pendant 20 min à 4°C. On récupère le culot et on l'homogénéise à l'aide d'un broyeur Polytron™ dans 15 volumes d'eau bidistillée à 4°C, puis on le centrifuge à 8000 g pendant 20 min. On élimine le culot et on centrifuge le surnageant et la couche de peau ("buffy coat") à 40000 g pendant 20 min, on récupère le culot, on le remet en suspension dans 15 ml d'eau bidistillée et on le centrifuge encore une fois à 40000 g avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de [³H]-cytisine à 1 nM dans un volume final de 500 µl de tampon, en présence ou en absence de composé à tester. On arrête la réaction par filtration sur des filtres Whatman GF/B™ préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [³H]-cytisine, puis on calcule la CI₅₀, concentration de composé qui inhibe 50% de la liaison spécifique.

Les CI50 des composés de l'invention les plus affins se situent entre 1,4 et 4 µM.

Les résultats qui précèdent montrent que les composés de l'invention sont des ligands sélectifs pour les sous-unités α₇ par rapport aux sous-unités α₄β₂ du récepteur nicotinique.

Les résultats des divers essais suggèrent l'utilisation des composés dans le traitement ou la prévention des désordres liés à un dysfonctionnement des récepteurs nicotiniques, notamment au niveau du système nerveux central.
Ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multiinfarct dementia, MDI).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des accidents vasculaires cérébraux et des épisodes hypoxiques cérébraux. Ils peuvent être utilisés dans les cas de pathologies psychiatriques : schizophrénie, dépression, anxiété, attaques de panique, comportements compulsifs et obsessionnels.
Ils peuvent prévenir les symptomes dus au sevrage au tabac, à l'alcool, aux différentes substances induisant une dépendance, telles que cocaïne, LSD, cannabis, benzodiazépines.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.
Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé répondant à la formule générale (I) 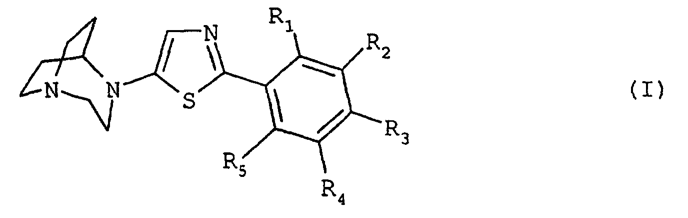 dans laquelle
R₁, R₂, R₃, R₄ et R₅ représentent chacun, indépendamment les uns des autres, un atome d'hydrogène ou d'halogène ou un groupe nitro, amino, trifluorométhyle, trifluoroalcoxy, cyano, hydroxy, (C₁-C₆) alkyle ou (C₁-C₆) alcoxy, à l'état de base ou de sel d'addition à un acide.

2. Procédé de préparation d'un composés selon la revendication 1, **caractérisé en ce qu'**on fait réagir le 1,4-diazabicyclo[3.2.2]nonane avec un composé de formule générale (III) 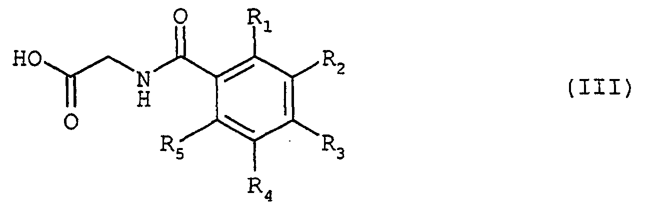 dans laquelle R₁, R₂, R₃, R₄ et R₅ sont tels que définis ci-dessus, puis on effectue une cyclisation de ce dernier en présence de dimère de sulfure de 4-méthoxyphénylthionophosphine (réactif de Lawesson), ou bien en présence de 2,4-disulfure de 2,4-bis(phénylthio)1,3,2,4-dithiadiphosphetane.

3. Médicament **caractérisé en ce qu'**il consiste en un composé selon la revendication 1.

4. Composition pharmaceutique, **caractérisée en ce qu'**elle contient un composé selon la revendication 1, associé à un excipient.

## Patentansprüche

1. Verbindung der allgemeinen Formel (I) 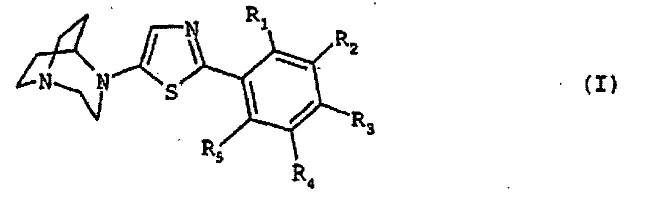 in der
R₁, R₂, R₃, R₄ und R₅ jeweils unabhängig voneinander ein Wasserstoffatom oder ein Halogenatom oder eine Nitro-, Amino-, Trifluormethyl-, Trifluoralkoxy-, Cyano-, Hydroxy-, (C₁-C₆)-Alkyl- oder (C₁-C₆)-Alkoxygruppe bedeuten, in Form der Base oder der Säureadditionssalze.

2. Verfahren zur Herstellung einer Verbindung nach Anspruch 1, **dadurch gekennzeichnet, daß** man 1,4-Diazabicyclo[3.2.2]nonan mit einer Verbindung der allgemeinen Formel (III) umsetzt 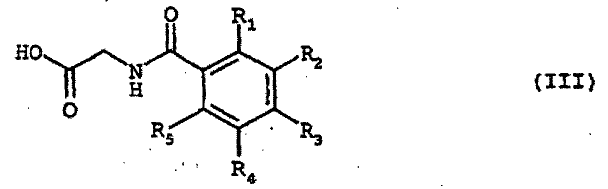 in der R₁, R₂, R₃, R₄ und R₅ die oben angegebenen Bedeutungen besitzen, dann eine Cyclisierung der erhaltenen Verbindung in Gegenwart des Dimeren des 4-Methoxyphenylthionophosphin-sulfids (Lawesson-Reagens) oder in Gegenwart des 2,4-Disulfids von 2,4-Bis(phenylthio)-1,3,2,4-dithiadiphosphetan bewirkt.

3. Arzneimittel, **dadurch gekennzeichnet, daß** es aus einer Verbindung nach Anspruch 1 besteht.

4. Pharmazeutische Zubereitung, **dadurch gekennzeichnet, daß** sie eine Verbindung nach Anspruch 1 in Kombination mit einem Trägermaterial enthält.

## Claims

1. Compound corresponding to the general formula (I) 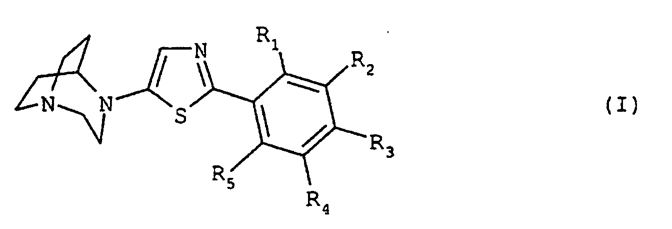 in which
R₁, R₂, R₃, R₄ and R₅ each represent, independently of each other, a hydrogen or halogen atom or a nitro, amino, trifluoromethyl, trifluoroalkoxy, cyano, hydroxyl, (C₁-C₆) alkyl or (C₁-C₆)alkoxy group, in the form of a base or of an addition salt with an acid.

2. Process for preparing a compound according to Claim 1, **characterized in that** 1,4-diazabicyclo[3.2.2]nonane is reacted with a compound of general formula (III) 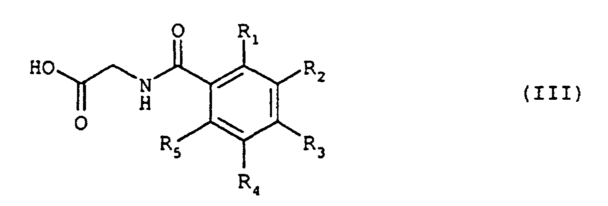 in which R₁, R₂, R₃, R₄ and R₅ are as defined above, and the above product is then cyclized in the presence of 4-methoxyphenylthionophosphine sulphide dimer (Lawesson's reagent), or alternatively in the presence of 2,4-bis(phenylthio)-1,3,2,4-dithiadiphosphetane 2,4-disulphide.

3. Medicinal product, **characterized in that** it consists of a compound according to Claim 1.

4. Pharmaceutical composition, **characterized in that** it contains a compound according to Claim 1, combined with an excipient.
